# EUROPEAN PATENT SPECIFICATION

(11) **EP 3 724 201 B1**
(45) Date of publication and mention of the grant of the patent: **02.02.2022**
(21) Application number: 18842490.7
(22) Date of filing: 11.12.2018
(51) Int. Cl.: C07F 9/655, A61K 8/67, A61K 8/19, A61Q 11/00

(54) **NOVEL COMPOUNDS**
NEUARTIGE VERBINDUNGEN
NOUVEAUX COMPOSÉS

(30) Priority: 15.12.2017 US 201762599077 P
(43) Date of publication of application: 21.10.2020
(73) Proprietor: Colgate-Palmolive Company, New York, NY 10022 (US)
(72) Inventor: HAO, Zhigang, Bridgewater New Jersey 08807 (US); CHENG, Chi-Yuan, Hillsborough New Jersey 08844 (US); PAN, Long, Somerset New Jersey 08873 (US); SUBRAMANYAM, Ravi, Belle Mead New Jersey 08502 (US)
(74) Representative: Wibbelmann, Jobst
(86) International application number: PCT/US2018/064940
(87) International publication number: WO 2019/118452

(56) References cited:
- WO-A1-2010/131994

## Description

### BACKGROUND

Oral care compositions which contain stannous ion sources exhibit excellent clinical benefits, particularly in the reduction of gingivitis and in the treatment or prevention of erosive tooth demineralization. Stannous fluoride is well known for use in clinical dentistry with a history of therapeutic benefits over forty years. However, until recently, its popularity has been limited by its instability in aqueous solutions. This restricts the range of formulations into which stannous compounds, for example, may be incorporated. The instability of stannous fluoride in water is primarily due to the reactivity of the stannous ion (Sn²⁺). Stannous salts readily hydrolyse above a pH of 4, resulting in precipitation from solution, with a consequent loss of the therapeutic properties.

One way to overcome the stability problems with stannous ions is to limit the amount of water in the composition to very low levels, or to use a dual phase system. Both of these solutions to the stannous ion problem have drawbacks. Low water oral care compositions can be difficult to formulate with desired rheological properties, and dual-phase compositions are considerably more expensive to manufacture and package.

Accordingly, in view of the drawbacks and disadvantages to using various antimicrobials, such as stannous, there is a need for oral care compositions with anti-bacterial efficacy, but which are also palatable and desirable for a user and which have improved solubility and stability. WO2010/131994 discloses in claim 9 the use of magnesium ascorbyl phosphate in the treatment of gingivitis.

### BRIEF SUMMARY

It has now been discovered that stannous and stannic ions can form a complex with ascorbyl phosphate. Without being bound by theory, Applicants believe that in one aspect this complex can possesses a special structural bonding system. In one aspect, Applicants believe that the stannous is not only bonded with the phosphate group like other phosphate complex (i.e., tetrasodium pyrophosphate (TSPP)) but is also bonded to the hydroxyl group on the ascorbyl portion in the structure. It is believed that in at least one aspect the complexes disclosed herein can exhibit both stability and solubility for at least Sn(II) ion in aqueous media system around neutral pH. Based on the fundamental understanding of stannous efficacy, and without being bound by any theory, it is believed that it is possible that the complexes described here, e.g., stannous ascorbyl phosphate, can deliver a relatively stronger stannous activity than certain market products.

The complex may be stannous-ascorbyl phosphate (SAP2) or stannic-ascorbyl phosphate (SAP4). The complex comprising stannous or stannic in arrangement with ascorbyl phosphate. In one aspect the ascorbyl phosphate is derived from sodium ascorbyl phosphpate. Without being bound by any theory, when placed in an oral care formulation, this complex provides an effective concentration of stannous or stannic ions for antibacterial activity, for example, delivering an antibacterial effective amount of stannous or stannic to the tooth enamel, as well as delivering an effective amount to protect against erosion and reducing bacterial colonization and biofilm development. Also, without being bound by theory, it is believed that the formation of the complex allows the stannous or stannic to avoid being oxidized too quickly (i.e., as compared to stannous or stannic not in complex with ascorbyl phosphate). Therefore, Applicants submit that the complexes described herein may have increased stability and improved delivery of stannous or stannous to the tooth enamel or gums.

The invention thus provides oral care compositions, for example, mouthwash, oral gel or dentifrice compositions, that comprise a complex that comprises a stannous-ascorbyl phosphate complex or stannic-ascorbyl phosphate complex. The compositions may optionally further comprise a fluoride source and or an additional phosphate source. The compositions may optionally further comprise a zinc source and/or an amino acid source. The compositions may be formulated in a suitable oral care formulation e.g., a conventional dentifrice, oral gel or mouthwash base, e.g., comprising one or more abrasives, surfactants, foaming agents, vitamins, polymers, enzymes, humectants, thickeners, antimicrobial agents, preservatives, flavorings, and/or colorants.

Disclosed are oral care methods of using the compositions of the invention to reduce and inhibit acid erosion of the enamel, clean the teeth, reduce bacterially-generated biofilm and plaque, reduce gingivitis, inhibit tooth decay and formation of cavities, and reduce dentinal hypersensitivity, comprising applying a composition of the invention to the teeth.

The invention further provides methods of making the compositions of the invention comprising combining a stannic or stannous ion source (e.g., stannous fluoride), a source of ascorbyl phosphate in aqueous solution, optionally isolating the complex thus formed as a solid; and admixing with an oral care base.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

Figure 1 shows the mass spectra of SAP2 complex.
Figure 2 shows the SAP2 structure in mass spectrum and stannous isotopic ratio.
Figure 3 shows the mass spectra of SAP4 complex.
Figure 4 shows the SAP4 structure in mass spectrum and stannous isotopic ratio.
Figure 5. Structural confirmation of SAP2 in water by ¹³C-NMR spectroscopy.

The top spectrum depicts the synthetic SAP2 and the bottom spectrum depicts sodium ascorbyl phosphate standard.

### DETAILED DESCRIPTION

The following description of the preferred embodiment(s) is merely exemplary in nature and is in no way intended to limit the invention, its application, or uses.

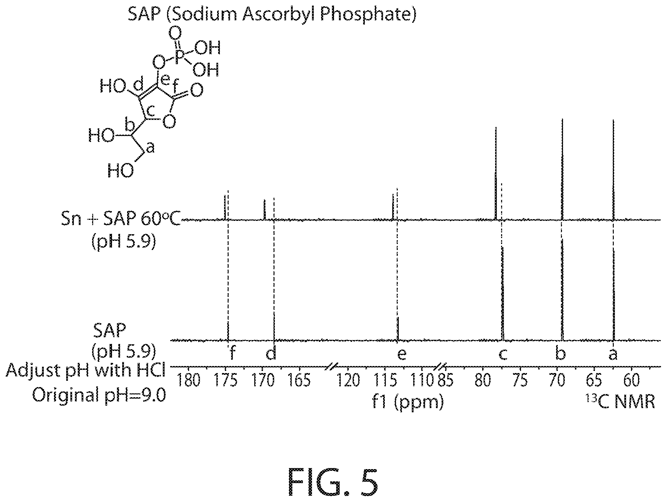

The invention therefore provides, in a first aspect, a stannous (SnII) ascorbyl phosphate complex (Complex 1) (SAP2). For example, Complex 1 can include:
1.1 Complex 1, wherein the stannous center is divalent stannous.
1.2 Any preceding complex, wherein the complex comprises stannous and ascorbyl phosphate in a 3:1 to 1:3 molar ratio, e.g., a 1:1 molar ratio.
1.3 Any preceding complex, wherein the complex has a three-dimensional structure wherein the stannous center is coordinated to three of the ascorbyl phosphate oxygen atoms.
1.4 Any preceding complex, wherein the complex shows as its major mass spectral peaks ions of m/z 372.9, 370.9, 368.9, 371.9, 369.9, 376.9, 374.9, 373.9, 371.9 (each +/- 14).
1.5 Any preceding complex, wherein the complex has the ¹³C-NMR spectrum shown in Figure 5.
1.6 Any preceding complex, wherein the complex is formed by combining a cationic ion-ascorbyl phosphate group with a source of stannous.
1.7 The complex of 1.6, wherein the cationic ion is selected from: aluminum, ammonium, calcium, copper, magnesium, sodium and potassium, and wherein the stannous source is selected from stannous chloride, stannous pyrophosphate, stannous nitric, stannous sulfate, and stannous fluoride.
1.8 Any preceding complex, wherein the complex is formed by combining sodium ascorbyl phosphate and stannous fluoride.
1.9 Any of the preceding complexes, wherein the complex forms in-situ in an oral care composition upon admixture of the stannous fluoride and sodium ascorbyl phosphate.
1.10 Any of the preceding complexes further comprising a basic amino acid.
1.11 Any of the preceding complexes wherein the amino acid is arginine or lysine or salts thereof.
1.12 Any of the preceding complexes wherein the basic amino acid is arginine.
1.13 Any of the preceding complexes wherein the complex further comprises a zinc ion source.
1.14 Any of the preceding complexes wherein the zinc ion source is selected from zinc citrate and/or zinc oxide.

The invention therefore provides, in a second aspect, a stannic (SnIV) ascorbyl phosphate complex (Complex 2) (SAP4). For example, Complex 2 can include:
2.1 Complex 2, wherein the stannic center is tin (IV).
2.2 Any preceding complex, wherein the complex comprises stannic and ascorbyl phosphate in a 3:1 to 1:3 molar ratio, e.g., a 1:1 molar ratio.
2.3 Any of the preceding complexes, wherein the complex has a three-dimensional structure wherein the stannic center is coordinated with one of the ascorbyl phosphate oxygen atoms.
2.4 Any of the preceding complexes, wherein the complex shows as its major mass spectral peaks ions of m/z 450.9, 448.9, 446.9, 449.9, 447.9, 454.9, 452.9 (each +/- 12)
2.5 Any preceding complex, wherein the complex is formed by combining a cationic ion-ascorbyl phosphate group with a source of stannic.
2.6 The complex of 2.5, wherein the cationic ion is selected from: aluminum, ammonium, calcium, copper, magnesium, sodium and potassium, and wherein the stannous source is selected from: stannous chloride, stannic oxide, stannic fluoride, and stannic ammonium chloride.
2.7 Any of the preceding complexes, wherein the complex is formed by combining sodium ascorbyl phosphate and stannic fluoride.
2.8 Any of the preceding complexes, wherein the complex forms in-situ in an oral care composition upon admixture of the stannic fluoride and sodium ascorbyl phosphate.
2.9 Any of the preceding complexes further comprising a basic amino acid.
2.10 Any of the preceding complexes wherein the amino acid is arginine or lysine or salts thereof.
2.11 Any of the preceding complexes wherein the basic amino acid is arginine.
2.12 Any of the preceding complexes wherein the complex further comprises a zinc ion source.
2.13 Any of the preceding complexes wherein the zinc ion source is selected from zinc citrate and/or zinc oxide.

The invention therefore provides, in a third embodiment, an oral care composition (Composition 1), comprising any of Complex 1, et seq, or Complex 2, et seq., e.g.,
1.1 Composition 1, wherein any of Complex 1 (i.e., SAP2), *et seq,* or Complex 2 (i.e., SAP4), *et seq.,* is present in an amount to provide the stannous or stannic in an amount of 0.05 to 10% by weight of the composition, optionally at least 0.1, at least 0.2, at least 0.3, at least 0.4, at least 0.5, at least 1, at least 2, at least 3, or at least 4 up to 10% by weight of the composition, e.g. about 1-3%, e.g., about 2-2.7% by weight.
1.2 Composition 1 or 1.1, wherein any of Complex 1, *et seq,* or Complex 2, *et seq.,* is present in an amount to provide the stannic or stannous ions in an amount of 0.05 to 30% by weight of the composition, optionally at least 0.1, at least 0.2, at least 0.3, at least 0.4, at least 0.5, at least 1, at least 2, at least 3, at least 4, at least 5, at least 10, at least 15, at least 20 up to 30% by weight, e.g., about 1-10% by weight.
1.3 Composition 1, 1.1 or 1.2, wherein any of Complex 1, *et seq,* or Complex 2, *et seq.,* is present in an amount to provide the ascorbyl phosphate in an amount of 0.05 to 20% by weight of the composition, optionally at least 0.1, at least 0.2, at least 0.3, at least 0.4, at least 0.5, at least 1, at least 2, at least 3, at least 4, at least 5, at least 10, or at least 15, up to 20% by weight, e.g., about 1-10% by weight.
1.4 Any of the foregoing compositions further comprising a fluoride ion source, wherein the fluoride ion source provides an effective amount of fluoride, e.g., providing 500 to 3000 ppm fluoride.
1.5 Any of the foregoing compositions further comprising an effective amount of fluoride, e.g., wherein the fluoride source is selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and combinations thereof.
1.6 Any of the preceding compositions comprising an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these, e.g., in an amount of 1-20%, e.g., 2-8%, e.g., ca. 5%, by weight of the composition.
1.7 Any of the foregoing compositions comprising buffering agents, e.g., sodium phosphate buffer (e.g., sodium phosphate monobasic and disodium phosphate).
1.8 Any of the foregoing compositions comprising a humectant, e.g., selected from glycerin, sorbitol, propylene glycol, polyethylene glycol, xylitol, and mixtures thereof, e.g. comprising at least 20%, e.g., 20-40%, e.g., 25-35% glycerin.
1.9 Any of the preceding compositions comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS); and/or a zwitterionic surfactant, for example a betaine surfactant, for example cocamidopropylbetaine, e.g. in an amount of from about 0.1% to about 4.5% by weight, e.g. 0.5-2% cocamidopropylbetaine.
1.10 Any of the preceding compositions further comprising a viscosity modifying amount of one or more of polysaccharide gums, for example xanthan gum or carrageenan, silica thickener, and combinations thereof.
1.11 Any of the preceding compositions further comprising flavoring, fragrance and/or coloring.
1.12 Any of the foregoing compositions further comprising a cosmetically acceptable carrier comprising one or more ingredients selected from water-soluble alcohols (such as C₂₋₈ alcohols including ethanol); glycols (including propylene glycol, dipropylene glycol, tripropylene glycol and mixtures thereof); glycerides (including mono-, di- and triglycerides); medium to long chain organic acids, alcohols and esters; surfactants (including emulsifying and dispersing agents); additional amino acids; structurants (including thickeners and gelling agents, for example polymers, silicates and silicon dioxide); emollients; fragrances; and colorants (including dyes and pigments).
1.13 Any of the foregoing compositions further comprising an effective amount of one or more antibacterial agents, for example comprising an antibacterial agent selected from: herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.14 Any of the preceding compositions further comprising a whitening agent, e.g., a whitening agent selected from the group consisting of peroxides, metal chlorites, perborates, percarbonates, peroxyacids, hypochlorites, and combinations thereof.
1.15 Any of the foregoing compositions further comprising an anionic polymer, e.g., a synthetic anionic polymeric polycarboxylate, e.g., wherein the anionic polymer is selected from 1:4 to 4:1 copolymers of maleic anhydride or acid with another polymerizable ethylenically unsaturated monomer; e.g., wherein the anionic polymer is a methyl vinyl ether/maleic anhydride (PVM/MA) copolymer having an average molecular weight (M.W.) of about 30,000 to about 1,000,000, e.g., about 300,000 to about 800,000, e.g., wherein the anionic polymer is about 1-5%, e.g., about 2%, of the weight of the composition.
1.16 Any of the preceding compositions further comprising a basic amino acid, wherein the basic amino acid is arginine or salts thereof
1.17 Any of the preceding compositions wherein the basic amino acid is arginine, and wherein the arginine is present in an amount corresponding to 1% to 15%, e.g., 3 wt. % to 10 wt. % of the total composition weight, about e.g., 1.5%, 4%, 5%, or 8%, wherein the weight of the basic amino acid is calculated as free form.
1.18 Any of the preceding compositions wherein the amino acid is arginine from 0. 1 wrt. % - 6.0 wt. %. (e.g., about 1.5 wt.%).
1.19 Any of the preceding compositions wherein the amino acid is arginine from about 1.5 wt. %.
1.20 Any of the preceding compositions, wherein composition further comprises a source of zinc, and wherein the source of zinc comprises zinc oxide and zinc citrate.
1.21 Any of the preceding compositions, wherein the ratio of the amount of zinc oxide (e.g., wt.%) to zinc citrate (e.g., wt.%) is from 1.5:1 to 4.5:1 (e.g., 2:1, 2.5:1, 3:1, 3.5:1, or 4:1).
1.22 Any of the preceding compositions, wherein the zinc citrate is in an amount of from 0.25 to 0.75 wt% (e.g., 0.5 wt. %) and zinc oxide may be present in an amount of from 0.75 to 1.25 wt% (e.g., 1.0 wt. %) based on the weight of the oral care composition.
1.23 Any of the preceding compositions wherein the zinc citrate is about 0.5 wt.%.
1.24 Any of the preceding compositions wherein the zinc oxide is about 1.0 wt.%.
1.25 Any of the preceding compositions where the zinc citrate is about 0.5 wt.% and the zinc oxide is about 1.0 wt.%).
1.26 Any of the preceding compositions further comprising a breath freshener, fragrance or flavoring.
1.27 Any of the foregoing compositions, wherein the pH of the composition is approximately neutral, e.g., from pH 6 to pH 9 e.g., from pH 7 to pH 9, or from pH 7 to pH 8, or from pH 7 to pH 7.5, or from pH 7.5 to pH 8, or about pH 7.5.
1.28 Any of the preceding compositions, wherein the pH is from pH 6 to pH 8 (e.g., about pH 6, about pH 6.5, or about pH 7).
1.29 Any of the preceding compositions comprising from 5% - 50%, e.g., 10% - 50%, e.g., about 15%, 25%, 30%, and 35% water.
1.30 Any of the foregoing compositions wherein the composition is an oral care composition is selected from a group consisting of: dentifrice, toothpaste, tooth powder, gel, and mouthwash.
1.31 Any of the forgoing compositions for use to reduce and inhibit acid erosion of the enamel, clean the teeth, reduce bacterially-generated biofilm and plaque, reduce gingivitis, inhibit tooth decay and formation of cavities, and reduce dentinal hypersensitivity.

Disclosed is a method to reduce and inhibit acid erosion of the enamel, clean the teeth, reduce bacterially-generated biofilm and plaque, reduce gingivitis, inhibit tooth decay and formation of cavities, and reduce dentinal hypersensitivity, comprising applying an effective amount of a complex of the invention, e.g., any of Complex 1, *et seq.,* or any of Complex 1, *et seq,* or a composition of the invention, e.g., any of Composition 1, *et seq.* to the teeth or oral cavity of a person in need thereof.

The invention further provides a method of making a complex of the invention, e.g., any of Complex 1 (SAP2), *et seq.,* or Complex 2 (SAP4) *et seq.,* the method comprising the steps of combining stannous fluoride or stannic fluoride, and sodium ascorbyl phosphate, in a suitable solvent, adjusting the pH, and isolating the resulting product, optionally as a solid salt. The temperature of the reaction is preferably maintained at between 30 °C and 70 °C, e.g., about 60 °C. Wherein the resulting pH range is between 5-8, e.g., about pH 7 for any of Complex 1 (SAP2), *et seq.,* and about pH 6.5 for any of Complex 2 (SAP4) *et seq.*

The invention further provides a method of making a composition of the invention, e.g., any of Composition 1, *et seq.,* comprising adding a purified complex of the invention, e.g., any of Complex 1, et seq., or Complex 2, et seq., to a suitable oral care. In some embodiments, the oral care is a dentifrice or mouthwash base.

Disclosed are methods to (i) reduce hypersensitivity of the teeth, (ii) reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; (xv) reduce tartar build-up, and/or (xvi) promote systemic health, including cardiovascular health, e.g., by reducing potential for systemic infection via the oral tissues, comprising applying any of Complex 1, et seq., of any of Complex 2 et seq., or any of Composition 1, et seq. as described above, to the oral cavity of a person in need thereof, e.g., one or more times per day. The invention further provides Complex 1, et seq., Complex 2, et seq., and Compositions 1, et seq. for use in any of these methods.

It will be understood that although the stannous, stannic, or ascorbyl phosphate may be present in a composition (e.g., an oral care composition) primarily in the form of precursor materials (e.g., stannous fluoride, stannic fluoride, or sodium ascorbyl phosphate) or in the form of the Complex of the invention, e.g., any of Complex 1 (SAP2) et seq., or Complex 2 (SAP4) et seq., there may be some degree of equilibrium, so that the proportion of the material which is actually in complex compared to the proportion in precursor form may vary depending on the precise conditions of formulation, concentration of materials, pH, presence or absence of water, presence or absence of other charged molecules, and so forth.

Oral care compositions of the invention, e.g., any of Composition 1, *et seq,* include any oral care formulation known in the art, for example a toothpaste, gel, mouthwash, powder, cream, strip, gum, or any other known in the art.

The benefits of the oral care compositions of the invention are numerous. Without being bound by theory, the complex of stannous or stannic, with ascorbyl phosphate, is believed to have the ability to reduce the potential of the stannic or stannous forming precipitates in solution thereby reducing their effectiveness - i.e., which may occur with traditional sources such as stannous fluoride. By providing stannous/stannic containing compounds that can release stannous or stannic ions in oral cavities, the oral care compositions of the invention provide antimicrobial, antiplaque, antigingivitis, anticaries, and anticalculus benefits. Additional benefits include the treatment or prevention of erosive tooth demineralization.

In certain embodiments, the Compositions described herein, i.e., Compositions 1, et seq., comprise the stannous-ascorbyl phosphate or stannic-ascorbyl phosphate complexes in an amount of 0.05 to 10% by weight of the composition. In certain embodiments, precursors, e.g., stannous fluoride, stannic fluoride, or sodium ascorbyl phosphate are present in amounts such that when combined into the composition to form the stannous-ascorbyl phosphate or stannic-ascorbyl phosphate complex *in situ,* said complex would be present in an amount of 0.05 to 10 % by weight of the composition. In either of these embodiments, the amount of the complex can be varied for the desired purpose, such as a dentifrice or a mouthwash. In other embodiments, the amount of the complex is at least 0.1, at least 0.2, at least 0.3, at least 0.4, at least 0.5, at least 1, at least 2, at least 3, or at least 4 up to 10% by weight of the composition. In other embodiments, the amount of the complex is less than 9, less than 8, less than 7, less than 6, less than 5, less than 4, less than 3, less than 2, less than 1, less than 0.5 to 0.05 % by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

In certain embodiments, the composition can be anhydrous. By anhydrous, it is meant that there is less than 5% by weight water, optionally less than 4, less than 3, less than 2, less than 1, less than 0.5, or less than 0.1% water, down to 0%, by weight water.

When provided in an anhydrous composition, precursors, e.g., stannous fluoride, stannic fluoride, or ascorbyl phosphate may not significantly react to form the complex of the invention. When contacted with a sufficient amount of water, which can be in the form of saliva and/or water used to rinse the mouth during or after application of the composition, the precursors will react to form the complex of the invention.

The carrier represents all other materials in the composition other than the stannous ascorbyl phosphate or stannic ascorbyl phosphate complex or its precursors. The amount of carrier is then the amount to reach 100% by adding to the weight of the stannous-ascorbyl phosphate or stannic-ascorbyl phosphate, including any precursors.

*Oral Care Active Agents*: The compositions of the invention may comprise various agents which are active to protect and enhance the strength and integrity of the enamel and tooth structure and/or to reduce bacteria and associated tooth decay and/or gum disease, including or in addition to the stannous- ascorbyl phosphate or stannic - ascorbyl phosphate complexes. Effective concentration of the active ingredients used herein will depend on the particular agent and the delivery system used. It is understood that a toothpaste for example will typically be diluted with water upon use, while a mouth rinse typically will not be. Thus, an effective concentration of active in a toothpaste will ordinarily be 5-15× higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product.

*Fluoride Ion Source*: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counterion in the salt.

*Abrasives*: The compositions of the invention, e.g., Composition 1 et seq. include silica abrasives, and may comprise additional abrasives, e.g., a calcium phosphate abrasive, e.g., tricalcium phosphate ((Ca₃(PO₄)₂), hydroxyapatite (Ca₁₀(PO₄)₆(OH)₂), or dicalcium phosphate dihydrate (CaHPO₄ • 2H₂O), also sometimes referred to herein as DiCal or calcium pyrophosphate; calcium carbonate abrasive; or abrasives such as sodium metaphosphate, potassium metaphosphate, aluminum silicate, calcined alumina, bentonite or other siliceous materials, or combinations thereof.

Other silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 115 and 119. These silica abrasives are described in U.S. Pat. No. 4,340,583, to Wason. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention.

*Foaming agents:* The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Poluox^{®} is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. Where present, the amount of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants*: The compositions useful in the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate;
ii. higher alkyl sulfates, such as sodium lauryl sulfate;
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na);
iv. higher alkyl aryl sulfonates such as sodium dodecyl benzene sulfonate (sodium lauryl benzene sulfonate);
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Tartar control agents:* In various embodiments of the present invention, the compositions comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The invention thus may comprise phosphate salts. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of about 3-4% of the sodium phosphate dibasic and about 0.2-1% of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., about. 5-15%), by weight of the composition.

*Flavoring Agents*: The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight e.g. about 0.5 to about 1.5% by weight.

*Polymers*: The oral care compositions of the invention may also include additional polymers to adjust the viscosity of the formulation or enhance the solubility of other ingredients. Such additional polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water-soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water-soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The compositions of the invention may include an anionic polymer, for example, in an amount of from about 0.05 to about 5%. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g., as disclosed in U.S. Pat. No. 4,866,161 Sikes et al.

*Water:* The oral compositions may comprise significant levels of water. Water employed in the preparation of commercial oral compositions should be deionized and free of organic impurities. The amount of water in the compositions includes the free water which is added plus that amount which is introduced with other materials.

*Humectants:* Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment of the invention, the principal humectant is glycerin, which may be present at levels of greater than 25%, e.g. 25-35% about 30%, with 5% or less of other humectants.

*Other optional ingredients:* In addition to the above-described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

Unless stated otherwise, all percentages of composition components given in this specification are by weight based on a total composition or formulation weight of 100%.

Unless otherwise specifically identified, the ingredients for use in the compositions and formulations of the present invention are preferably cosmetically acceptable ingredients. By "cosmetically acceptable" is meant suitable for use in a formulation for topical application to human skin. A cosmetically acceptable excipient, for example, is an excipient which is suitable for external application in the amounts and concentrations contemplated in the formulations of this invention, and includes for example excipients which are "Generally Recognized as Safe" (GRAS) by the United States Food and Drug Administration.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

As used throughout, ranges are used as shorthand for describing each and every value that is within the range. Any value within the range can be selected as the terminus of the range.

In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### EXAMPLES

### Example 1: Stannous-Ascorbyl Phosphate and Stannic Ascorbyl Phosphate Synthesis

**Stannous ascorbyl phosphate sample preparation:** 3.220 g of sodium ascorbyl phosphate is dissolved into 100 mL water and then 1.567 g of SnF₂ is added. The mixture is stirred into completely solubilized and heat at 60C for one hour. The solution is directly analyzed by 13C-NMR and diluted into 5000ppm and analyzed by LC-MS

**Table 1. The material details for stannous ascorbyl phosphate synthesis**

| Stannous ascorbyl phosphate | | | | | |
|---|---|---|---|---|---|
| | MW | Amount (g) | mmols | CAS# | CF |
| Water | 18 | 100.000 | 5555.56 | | |
| sodium ascorbyl phosphate | 322.05 | 3.220 | 10.00 | 66170-10-3 | C₆H₆Na₃O₉P |
| Stannous Fluoride | 156.69 | 1.567 | 10.00 | 7783-47-3 | SnF2 |
| Sn-SAP% | | 4.568 | | | |

**Stannic ascorbyl phosphate sample preparation:** 0.644 g of SnF₂ sodium ascorbyl phosphate is dissolved into 100 mL water and then 1.567 g of SnF₄ is added. The mixture is stirred into completely solubilized and heat at 60C for one hour. The solution is directly analyzed by ¹³C-NMR and diluted into 5000ppm and analyzed by LC-MS.

**Table 2. The material details for stannic ascorbyl phosphate synthesis**

| Stannic ascorbyl phosphate | | | | | |
|---|---|---|---|---|---|
| | MW | Amount (g) | mmols | CAS# | CF |
| Water | 18 | 100.000 | 5555.56 | | |
| sodium ascorbyl phosphate | 322.05 | 0.644 | 2.00 | 66170-10-3 | C₆H₆Na₃O₉P |
| Stannic Fluoride | 194.7 | 0.390 | 2.00 | 7783-62-2 | SnF4 |
| Sn (IV) -SAP% | | 1.023 | | | |

### Example 2: LC-MS analysis

Briefly, LC-MS analysis is performed using a AB Sciex tandem mass spectrometer (AB Sciex LLC, Framingham, MA, USA) equipped with an ESI interface and Agilent 1260 capillary LC system (Model Agilent 1260, Agilent Technologies, Palo Alto, CA, USA). The capillary LC system is equipped with a capillary binary pump (Model G1376A), a DAD detector (G1315C), a micro vacuum degasser (Model G4225A), a thermostatted column compartment (Model G1316A. The capillary pump is set under the micro-flow mode. The LC separation is achieved by using an Agilent Zorbax SB-Aq column with 2.1 mm i.d.×50 mm dimension and 3.5 µm particle size (Agilent Technologies, Palo Alto, CA, USA Part No. 871700-914) . The mobile phase is methanol:water/5:95. The flow rate is 70 µL/min and the injected volume is 1 µL. The AB Sciex tandem mass spectrometer is operated in the negative-ion mode under the following conditions: nitrogen (>99.99%) is used for curtain gas at 10 psi, ion source gas 1 and 2 at 10 and 10 psi, respectively. ESI IonSpray voltage is set at 5.5 kV in ESI interface. The declustering and entrance potential are set up at 80 and 5.5 v, respectively. The temperature of the ionization interface is maintained at 550 °C. For total ion count (TIC) mode, the MS screen range is from 100 to 700 m/z. Data is acquired with an Analyst software 1.6.2 system (AB Sciex LLC, Framingham, MA, USA).

### Example 3: NMR experiment

¹³C NMR studies are performed on a Bruker Avance spectrometer (Bruker-Biospin, Billerica, MA, US) with a 5 mm CryoProdigy^{™} platform operating at 500.0 MHz for ¹H and 125.7 MHz for ¹³C in water at 25 °C. All ¹³C NMR spectra are acquired using a ¹H decoupling sequence ("zgig" from Bruker pulse-program library) with a repetition time of 15 sec and 4096 transients.

### Example 4: Computational Study

Computer calculation has been done at the B3LYP and M06 levels of density functional theory. Geometry optimizations and harmonic frequency calculation is performed at the B3LYP/BS1 level in aqueous solution using the SMD solvation model, BS1 designating a mixed basis set of SDD for zinc and 6-3 1G(d,p) for other atoms. The B3LYP/BS1-calculated harmonic frequencies is used to obtain zero-point energy-corrected Gibbs free energies at 298.15 K and 1 atm. The calculation is performed with Gaussian 09.

### Example 5: Detailed Experimental Results of the Invention

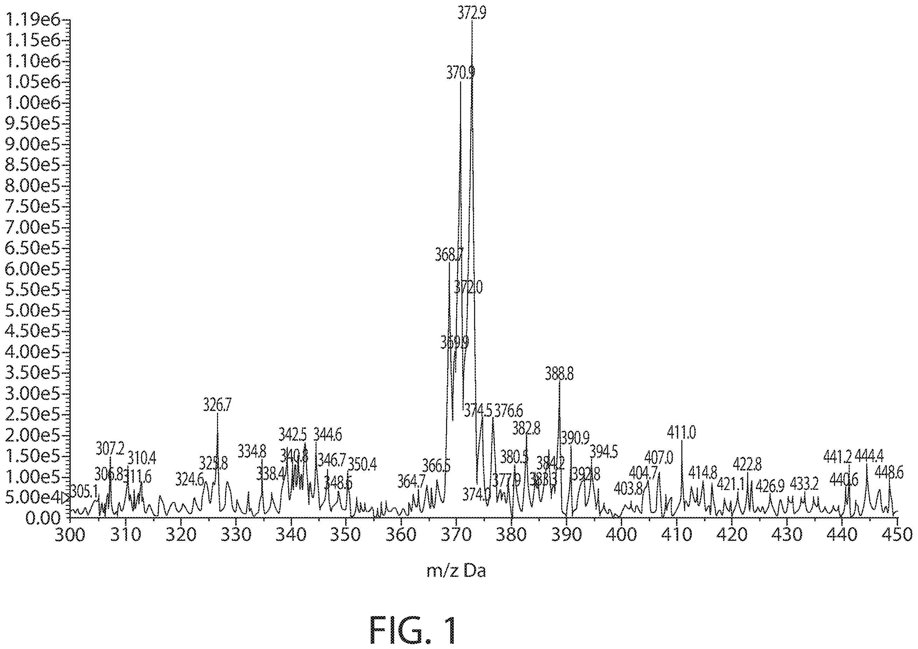

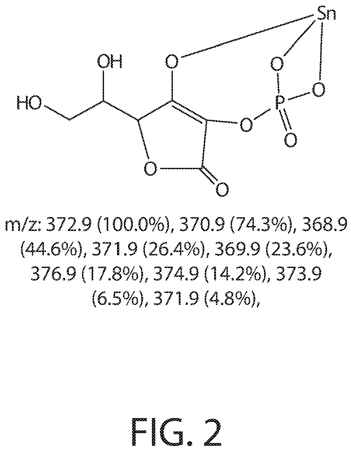

**Mass spectrometric (MS) results for stannous ascorbyl phosphate solution** (SAP2): Based on the raw materials added in table 1, the theoretical SAP2 concentration is 5% (w/w). This synthetic SAP2 solution is diluted to 5000 ppm with water, which is transferred into MS instrument for an analysis. The mass spectra of SAP2 are shown in figure 1. The stannous complex with the typical stannous isotopic pattern is identified at 372.9 (100%), 370.9 (74.3%), 368.9 (44.6%), 371.9 (26.4%), 369.9 (23.6%), 376.9 (17.8%) and 374.9 (14.2) cluster. As designed experimentally, the SAP2 mass spectra are matched with structure shown in figure 2 and the detailed isotope ratios obtained from ChemDraw software are also matched with mass spectra in figures 1 and 2.

### Example 6: Mass spectrometric (MS) results for stannic ascorbyl phosphate solution (SAP4)

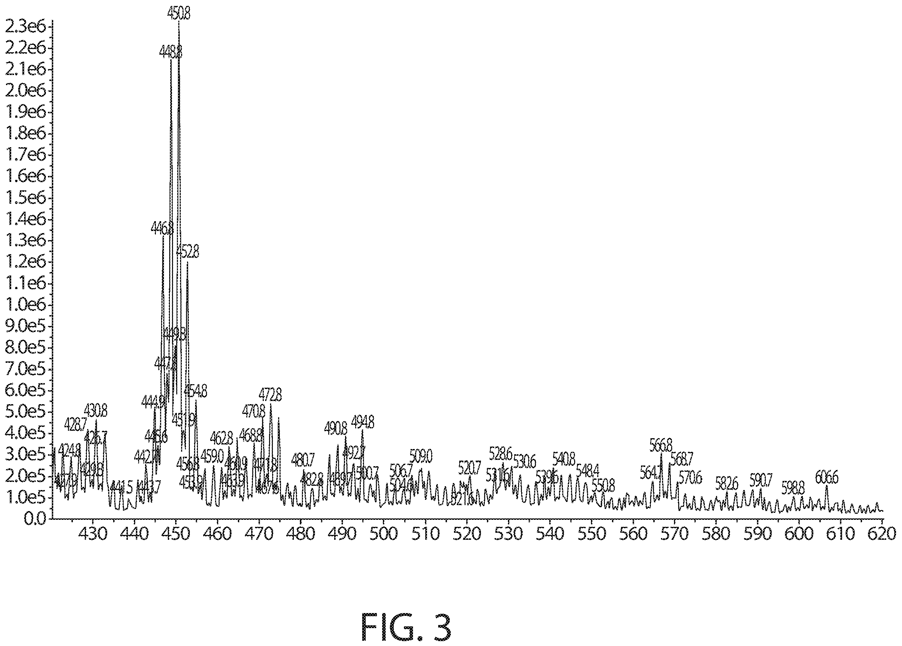

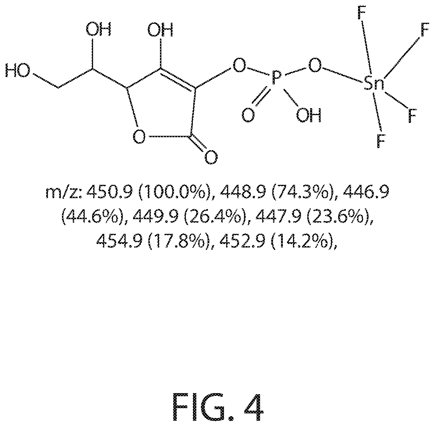

Based on the raw materials added in table 1, the theoretical SAP4 concentration is 1% (w/w). This synthetic SAP4 solution is diluted to 5000 ppm with water, which is transferred into MS instrument for an analysis. The mass spectra of SAP4 are shown in figure 3. The stannic complex with the typical stannic isotopic pattern is identified at 450.0 (100%), 448.9 (74.3%), 446.9 (44.6%), 449.9 (26.4%), 447.9 (23.6%), 454.9 (17.8%) and 452.9 (14.2%) cluster. As designed experimentally, the SAP4 mass spectra are matched with structure shown in figure 4 and the detailed isotope ratios obtained from ChemDraw software are also matched with mass spectra in figures 3 and 4.

### Example 7: Nuclear Magnetic Resonance (NMR) spectroscopic results for the synthetic SAP2 in water

The synthetic SAP2 (5%) in water solution is directly and slowly transferred into a 5mm NMR tube for an analysis. The ¹³C-NMR spectrum of such solution is shown in figure 3. The sodium ascorbyl phosphate dissolved in water and the pH is adjusted into 5.9 by adding 0.1 MHCl, which is same as SAP2 water solution, is present in figure 3 for comparison. The chemical shift of furan ring is different to sodium ascorbyl phosphate. These results suggest the stannous is attached onto hydroxyl group at d position in SAP2.

### Example 8: Density Functional Theory (DFT)

The DFT calculation provides a structure with minimal energy, in this structure, the stannous is coordinated to 3 oxygen atoms in which two from phosphate and one from ascorbic group. The coordination geometry of Stannous is a typical triangle pyramid, the apical position is occupied by a lone stannous pair.

### Example 9: Lack of Precipitate Formation

In one aspect, a solution of ~5% concentration of stannous ascorbyl phosphate is prepared as described in the above examples. It is expected that this stannous ascorbyl phosphate solution will not form an observable precipitate after three months following its preparation.

## Claims

1. A stannous complex, wherein the complex comprises a stannous (SnII) ascorbyl phosphate complex.

2. The complex of claim 1, wherein the stannous center is divalent stannous.

3. The complex of claim 1 or 2, wherein the complex comprises stannous and ascorbyl phosphate in a 3:1 to 1:3 molar ratio.

4. The complex of any of claims 1-3, wherein the complex has a three-dimensional structure and wherein the stannous center is coordinated to three of the ascorbyl phosphate oxygen atoms, and/or
wherein the complex shows as its major mass spectral peaks ions of m/z 372.9, 370.9, 368.9, 371.9, 369.9, 376.9, 374.9, 373.9, 371.9 (each +/- 14), and/or
wherein the complex has the ¹³C-NMR spectrum shown in Figure 5, and/or
wherein the complex is formed by combining sodium ascorbyl phosphate and stannous fluoride, and/or
wherein the complex forms in-situ in an oral care composition upon admixture of the stannous fluoride and sodium ascorbyl phosphate.

5. A stannic (SnIV) complex, wherein the complex comprises a stannic ascorbyl phosphate complex, optionally
wherein the stannic center is tin (IV).

6. A stannic complex of claim 5, wherein the complex comprises stannic and ascorbyl phosphate in a 3:1 to 1:3 molar ratio.

7. A stannic complex of claim 5 or 6, wherein the complex has a three-dimensional structure wherein the stannic center is coordinated with one of the ascorbyl phosphate oxygen atoms, and/or
wherein the complex shows as its major mass spectral peaks ions of m/z 450.9, 448.9, 446.9, 449.9, 447.9, 454.9, 452.9 (each +/- 12).

8. A stannic complex of any of claims 5-7, wherein the complex is formed by combining sodium ascorbyl phosphate and stannic fluoride, and/or
wherein the complex forms in-situ in an oral care composition upon admixture of the stannic fluoride and sodium ascorbyl phosphate.

9. An oral care composition comprising the stannous (SnII) ascorbyl phosphate complex and/or stannic (SnIV) ascorbyl phosphate complex of any of the preceding claims.

10. The oral care composition of claim 9, wherein the stannous (SnII) ascorbyl phosphate complex and/or stannic (SnIV) ascorbyl phosphate complex is present in an amount to provide the stannous or stannic in an amount of 0.05 to 10% by weight of the composition, and/or
wherein the stannous (SnII) ascorbyl phosphate complex and/or stannic (SnIV) ascorbyl phosphate complex is present in an amount to provide the ascorbyl phosphate in an amount of 0.05 to 20% by weight of the composition.

11. The oral care composition of claim 9 or 10, wherein the composition further comprises a fluoride source, optionally comprising an effective amount of a fluoride ion source providing between 500 to 3000 ppm fluoride.

12. The oral care composition of any of claims 9-11, wherein the composition further comprises a fluoride source selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.

13. The oral care composition of any of claims 9-12 further comprising a basic amino acid, wherein the amino acid is arginine or salts thereof, optionally
wherein the arginine is present in an amount corresponding to 1% to 15%.

14. The oral care composition of any of claims 9-13, further comprising a source of zinc, wherein the source of zinc comprises zinc oxide and zinc citrate, optionally
wherein the ratio of the amount of zinc oxide to zinc citrate is from 1.5:1 to 4.5:1.

15. The oral care composition of any of claims 9-14, wherein the zinc citrate is in an amount of from 0.25 to 0.75 wt% and zinc oxide may be present in an amount of from 0.75 to 1.25 wt% based on the weight of the oral care composition, optionally
wherein the zinc citrate is in an amount of about 0.5 wt% and the zinc oxide is in an amount of about 1.0 wt%.

## Patentansprüche

1. Zinnkomplex, wobei der Komplex einen Zinn(SnII)-Ascorbylphosphat-Komplex umfasst.

2. Komplex nach Anspruch 1, wobei das Zinnzentrum zweiwertiges Zinn ist.

3. Komplex nach Anspruch 1 oder 2, wobei der Komplex Zinn(II)- und Ascorbylphosphat in einem molaren Verhältnis von 3:1 bis 1:3 umfasst.

4. Komplex nach einem der Ansprüche 1 bis 3, wobei der Komplex eine dreidimensionale Struktur aufweist und das Zinnzentrum an drei der Ascorbylphosphat-Sauerstoffatome koordiniert ist, und/oder
wobei der Komplex als seine Hauptmassenspektralpeaks Ionen von m/z 372,9, 370,9, 368,9, 371,9, 369,9, 376,9, 374,9, 373,9, 371,9 (jeweils +/- 14) aufweist, und/oder wobei der Komplex das in Figur 5 gezeigte ¹³C-NMR-Spektrum aufweist, und/oder
wobei der Komplex durch Kombination von Natriumascorbylphosphat und Zinnfluorid gebildet wird, und/oder
wobei sich der Komplex in-situ in einer Mundpflegezusammensetzung durch Vermischen von Zinnfluorid und Natriumascorbylphosphat bildet.

5. Zinn(SnIV)-Komplex, wobei der Komplex einen Zinn-Ascorbylphosphat-Komplex umfasst, gegebenenfalls
wobei das Zinnzentrum Zinn (IV) ist.

6. Zinnhaltiger Komplex nach Anspruch 5, wobei der Komplex Zinnsäure und Ascorbylphosphat in einem molaren Verhältnis von 3:1 bis 1:3 umfasst.

7. Zinnkomplex nach Anspruch 5 oder 6, wobei der Komplex eine dreidimensionale Struktur aufweist, bei der das Zinnzentrum mit einem der Ascorbylphosphat-Sauerstoffatome koordiniert ist, und/oder
wobei der Komplex als seine Hauptmassenspektralpeaks Ionen von m/z 450,9, 448,9, 446,9, 449,9, 447,9, 454,9, 452,9 (jeweils +/- 12) aufweist.

8. Zinnhaltiger Komplex nach einem der Ansprüche 5 bis 7, wobei der Komplex durch Kombination von Natriumascorbylphosphat und Zinnfluorid gebildet wird, und/oder
wobei sich der Komplex in-situ in einer Mundpflegezusammensetzung bei der Beimischung von Zinnfluorid und Natriumascorbylphosphat bildet.

9. Mundpflegezusammensetzung, umfassend den Zinn(SnII)-Ascorbylphosphat-Komplex und/oder Zinn(SnIV)-Ascorbylphosphat-Komplex nach einem der vorhergehenden Ansprüche.

10. Mundpflegezusammensetzung nach Anspruch 9, wobei der Zinn(SnII)-Ascorbylphosphat-Komplex und/oder der Zinn(SnIV)-Ascorbylphosphat-Komplex in einer Menge vorhanden ist, um Zinn(II) oder Zinn(IV) in einer Menge von 0,05 bis 10 Gew.-% der Zusammensetzung bereitzustellen, und/oder
wobei der Zinn(SnII)-Ascorbylphosphat-Komplex und/oder der Zinn(SnIV)-Ascorbylphosphat-Komplex in einer Menge vorhanden ist, um das Ascorbylphosphat in einer Menge von 0,05 bis 20 Gew.-% der Zusammensetzung bereitzustellen.

11. Mundpflegezusammensetzung nach Anspruch 9 oder 10, wobei die Zusammensetzung ferner eine Fluoridquelle umfasst, die gegebenenfalls eine wirksame Menge einer Fluoridionenquelle umfasst, die zwischen 500 und 3000 ppm Fluorid liefert.

12. Mundpflegezusammensetzung nach einem der Ansprüche 9 bis 11, wobei die Zusammensetzung ferner eine Fluoridquelle umfasst, die ausgewählt ist aus Zinnfluorid, Natriumfluorid, Kaliumfluorid, Natriummonofluorphosphat, Natriumfluorosilicat, Ammoniumfluorosilicat, Aminfluorid (z.B., N'-Octadecyltrimethylendiamin-N,N,N'-tris(2-ethanol)-dihydrofluorid), Ammoniumfluorid, Titanfluorid, Hexafluorosulfat und Kombinationen davon.

13. Mundpflegezusammensetzung nach einem der Ansprüche 9-12, die ferner eine basische Aminosäure enthält, wobei die Aminosäure Arginin oder Salze davon ist, gegebenenfalls
wobei das Arginin in einer Menge von 1 % bis 15 % vorhanden ist.

14. Mundpflegezusammensetzung nach einem der Ansprüche 9-13, die ferner eine Zinkquelle umfasst, wobei die Zinkquelle Zinkoxid und Zinkcitrat umfasst, gegebenenfalls
wobei das Verhältnis der Menge an Zinkoxid zu Zinkcitrat von 1,5:1 bis 4,5:1 beträgt.

15. Mundpflegezusammensetzung nach einem der Ansprüche 9 bis 14, wobei das Zinkcitrat in einer Menge von 0,25 bis 0,75 Gew.-% und Zinkoxid in einer Menge von 0,75 bis 1,25 Gew.-%, bezogen auf das Gewicht der Mundpflegezusammensetzung, vorhanden sein kann, gegebenenfalls wobei das Zinkcitrat in einer Menge von etwa 0,5 Gew.-% und das Zinkoxid in einer Menge von etwa 1,0 Gew.-% vorhanden ist.

## Revendications

1. Complexe stanneux, dans lequel le complexe comprend un complexe de stanneux (SnII) phosphate d'ascorbyle.

2. Complexe selon la revendication 1, dans lequel le centre stanneux est un stanneux divalent.

3. Complexe selon la revendication 1 ou 2, dans lequel le complexe comprend un stanneux et du phosphate d'ascorbyle dans un rapport molaire de 3:1 à 1:3.

4. Complexe selon l'une quelconque des revendications 1 à 3, dans lequel le complexe a une structure tridimensionnelle et dans lequel le centre stanneux est coordonné à trois des atomes d'oxygène du phosphate d'ascorbyle, et/ou
dans lequel le complexe présente en tant que ses principaux pics de spectre de masse des ions de m/z 372,9, 370,9, 368,9, 371,9, 369,9, 376,9, 374,9, 373,9, 371,9 (chacun +/- 14), et/ou
dans lequel le complexe a le spectre ¹³C-RMN représenté sur la figure 5, et/ou
dans lequel le complexe est formé en combinant du phosphate d'ascorbyle de sodium et du fluorure stanneux, et/ou
dans lequel le complexe se forme in situ dans une composition de soins bucco-dentaires lors du mélange du fluorure stanneux et du phosphate d'ascorbyle de sodium.

5. Complexe stannique (SnIV), dans lequel le complexe comprend un complexe de stannique phosphate d'ascorbyle, éventuellement
dans lequel le centre stannique est l'étain (IV).

6. Complexe stannique selon la revendication 5, dans lequel le complexe comprend un stannique et du phosphate ascorbylique dans un rapport molaire de 3:1 à 1:3.

7. Complexe stannique selon la revendication 5 ou 6, dans lequel le complexe a une structure tridimensionnelle dans laquelle le centre stannique est coordonné avec l'un des atomes d'oxygène du phosphate d'ascorbyle, et/ou
dans lequel le complexe présente en tant que ses principaux pics de spectre de masse des ions de m/z 450,9, 448,9, 446,9, 449,9, 447,9, 454,9, 452,9 (chacun +/- 12).

8. Complexe stannique selon l'une quelconque des revendications 5 à 7, dans lequel le complexe est formé en combinant du phosphate d'ascorbyle de sodium et du fluorure stannique, et/ou
dans lequel le complexe se forme in situ dans une composition de soins bucco-dentaires lors du mélange du fluorure stannique et du phosphate d'ascorbyle de sodium.

9. Composition de soins bucco-dentaires comprenant le complexe de stanneux (SnII) phosphate d'ascorbyle et/ou le complexe de stannique (SnIV) phosphate d'ascorbyle selon l'une quelconque des revendications précédentes.

10. Composition de soins bucco-dentaires selon la revendication 9, dans laquelle le complexe de stanneux (SnII) phosphate d'ascorbyle et/ou le complexe de stannique (SnIV) phosphate d'ascorbyle est présent en une quantité pour fournir le stanneux ou le stannique en une quantité de 0,05 à 10 % en poids de la composition, et/ou
dans laquelle le complexe de stanneux (SnII) phosphate d'ascorbyle et/ou le complexe de stannique (SnIV) phosphate d'ascorbyle est présent en une quantité pour fournir le phosphate d'ascorbyle en une quantité de 0,05 à 20 % en poids de la composition.

11. Composition de soins bucco-dentaires selon la revendication ou 10, dans laquelle la composition comprend en outre une source de fluorure, comprenant éventuellement une quantité efficace d'une source d'ions fluorure fournissant entre 500 et 3 000 ppm de fluorure.

12. Composition de soins bucco-dentaires selon l'une quelconque des revendications 9 à 11, dans laquelle la composition comprend en outre une source de fluorure choisie parmi le fluorure stanneux, le fluorure de sodium, le fluorure de potassium, le monofluorophosphate de sodium, le fluorosilicate de sodium, le fluorosilicate d'ammonium, le fluorure d'amine (par exemple, le N'-octadécyltriméthylènediamine-N,N,N'-tris(2-éthanol)-dihydrofluorure), le fluorure d'ammonium, le fluorure de titane, l'hexafluorosulfate et leurs combinaisons.

13. Composition de soins bucco-dentaires selon l'une quelconque des revendications 9 à 12, comprenant en outre un acide aminé basique, dans laquelle l'acide aminé est l'arginine ou des sels cette dernière, éventuellement
dans laquelle l'arginine est présente en une quantité correspondant à 1 % à 15 %.

14. Composition de soins bucco-dentaires selon l'une quelconque des revendications 9 à 13, comprenant en outre une source de zinc, dans laquelle la source de zinc comprend de l'oxyde de zinc et du citrate de zinc, éventuellement
dans laquelle le rapport de la quantité d'oxyde de zinc au citrate de zinc est de 1,5:1 à 4,5:1.

15. Composition de soins bucco-dentaires selon l'une quelconque des revendications 9 à 14, dans laquelle le citrate de zinc est en une quantité de 0,25 à 0,75 % en poids et l'oxyde de zinc peut être présent en une quantité de 0,75 à 1,25 % en poids par rapport au poids de la composition de soins bucco-dentaires, éventuellement
dans laquelle le citrate de zinc est en une quantité d'environ 0,5 % en poids et l'oxyde de zinc est en une quantité d'environ 1,0 % en poids.
